(19) Europäisches Patentamt
European Patent Office
Office européen des brevets

(11) Veröffentlichungsnummer: **0 211 254**
**A2**

# (12) EUROPÄISCHE PATENTANMELDUNG

(21) Anmeldenummer: 86109315.1

(22) Anmeldetag: 08.07.86

(51) Int. Cl.⁴: **C 07 C 97/065**
**A 61 K 31/135**

(30) Priorität: 31.07.85 DE 3527355
29.08.85 DE 3530789

(43) Veröffentlichungstag der Anmeldung:
25.02.87 Patentblatt 87/9

(84) Benannte Vertragsstaaten:
AT BE CH DE FR GB IT LI LU NL SE

(71) Anmelder: Degussa Aktiengesellschaft
Weissfrauenstrasse 9
D-6000 Frankfurt am Main 1(DE)

(72) Erfinder: Engel, Jürgen, Dr.
Erlenweg 3
D-8755 Alzenau(DE)

(72) Erfinder: Kutscher, Bernhard, Dr.
Am Erlenbruch 16
D-6000 Frankfurt/Main 60(DE)

(72) Erfinder: Oepen, Gerhard, Dr.
Höriweg 4
D-7752 Insel Reichenau 1(DE)

(72) Erfinder: Niebch, Georg, Dr.
Südring 43
D-6458 Rodenbach(DE)

(72) Erfinder: Metzenauer, Peter, Dr.
Taunusstrasse 9
D-6466 Gründau 3(DE)

(54) **Neue cycloaliphatische Ketoamine.**

(57) Herzwirksame Verbindungen der Formel

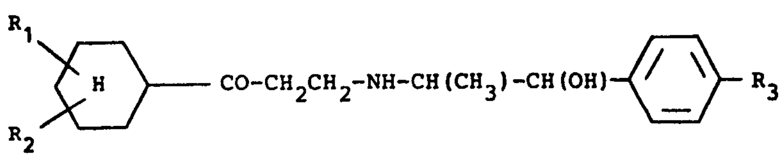

I

worin $R_1$ Wasserstoff, eine Hydroxygruppe oder eine $C_2$–$C_{13}$-Alkanoyloxygruppe, $R_2$ eine Hydroxygruppe oder eine $C_2$–$C_{13}$-Alkanoyloxygruppe und $R_3$ Wasserstoff, eine Hydroxygruppe, eine $C_2$–$C_{13}$-Alkanoyloxygruppe oder eine Benzyloxygruppe ist und deren Säureadditionssalze und Verfahren zu deren Herstellung.

EP 0 211 254 A2

0211254

D e g u s s a Aktiengesellschaft
Weissfrauenstraße 9, 6000 Frankfurt/Main

# Neue cycloaliphatische Ketoamine

In der deutschen Offenlegungsschrift 29 19 495 werden Verbindungen der Formel

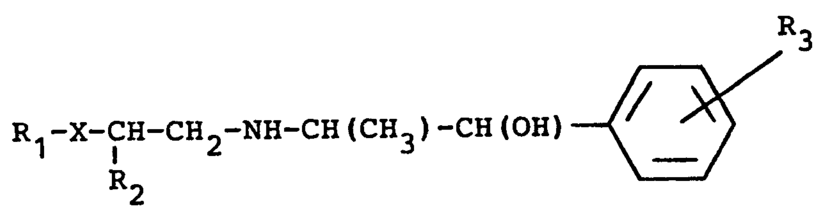

I

beschrieben, worin X die Gruppe >CO oder >CH(OH), $R_2$ Wasserstoff oder eine $C_1$-$C_6$-Alkylgruppe und $R_3$ Wasserstoff oder eine Hydroxygruppe ist und $R_1$ den Adamantylrest oder einen gesättigten oder einfach ungesättigten $C_3$-$C_{16}$-Cycloalkylrest bedeutet, wobei dieser $C_3$-$C_{16}$-Cycloalkylrest auch durch eine $C_1$-$C_4$-Alkylgruppe oder ein Halogenatom substituiert sein kann.
Diese Verbindungen verbessern die cerebrale oder periphere Durchblutung.

Die Erfindung betrifft neue Verbindungen der Formel I, Verfahren zu deren Herstellung und Arzneimittel, die diese Verbindungen als Wirkstoffe enthalten.

...

Die erfindungsgemäßen Verbindungen sind pharmakologisch wirksam und bewirken eine Verbesserung der Kontraktilität des Herzens ohne dabei die Frequenz und den Blutdruck zu beeinflussen.

Falls die Reste $R_1$, $R_2$ und/oder $R_3$ eine $C_2$-$C_{13}$-Alkanoyloxygruppe bedeuten, kann der Alkanoylteil dieser Gruppe gerade oder verzweigt sein. Vorzugsweise handelt es sich um $C_2$-$C_7$-Alkanoyloxygruppen. Die Reste $R_1$, $R_2$ und $R_3$ können gleich oder verschieden sein. Wenn die Reste $R_1$, $R_2$ und $R_3$ Alkanoyloxygruppen darstellen, kann es sich ebenfalls um gleiche oder verschiedene Alkanoyloxygruppen handeln.

Bei dem angegebenen Herstellungsverfahren ist es häufig zweckmäßig, in den Ausgangsstoffen die phenolische Hydroxylgruppe durch an sich bekannte Schutzgruppen zu schützen. Manchmal sind solche Schutzgruppen bereits schon für die Herstellung der Ausgangsverbindungen selbst erforderlich. Diese Schutzgruppen sind aus den Endprodukten leicht abspaltbar. Es handelt sich entweder um leicht solvolytisch abspaltbare Acylgruppen oder hydrierend abspaltbare Gruppen, wie zum Beispiel den Benzylrest. Die solvolytisch abspaltbaren Schutzgruppen werden beispielsweise durch Verseifung mit verdünnten Mineralsäuren in einem Lösungs- oder Suspensionsmittel (niedere Alkohole) bei Temperaturen zwischen 10 und 150° C abgespalten. Je nach Art der Schutzgruppe erfolgt bereits aber auch Abspaltung während der Verfahrensreaktion. Letzteres ist beispielsweise dann der Fall, wenn die phenolische Hydroxygruppe durch eine Benzylgruppe oder den Carbobenzoxyrest geschützt ist und das Verfahren einen Hydrierungsschritt enthält. Wird die Schutzgruppe nicht während der Reaktion abgespalten, so ist eine einfache Nachbehandlung des Reaktionsproduktes erforder-

...

lich, wobei dann die Abspaltung der Schutzgruppe beispielsweise durch Bedingungen wie sie oben angegeben sind, erfolgt.

Als Schutzgruppen kommen beispielsweise in Frage: Benzylgruppe, $\alpha$-Phenylethylgruppe, im Benzolkern substituierte Benzylgruppen wie zum Beispiel die p-Brom- oder p-Nitro-benzylgruppe, die Carbobenzoxygruppe, die Carbobenzthiogruppe, die Trifluoracetylgruppe, der Phthalylrest, der Tritylrest, der p-Toluolsulfonylrest und ähnliche, aber zusätzlich sind hier auch einfache Acylgruppen wie zum Beispiel die Acetylgruppe, Formylgruppe oder die tert.-Butylcarboxygruppe geeignet.

Vorhandene Hydroxygruppen von Verfahrensprodukten der Formel I können durch eine $C_2$-$C_{13}$-Alkanoylgruppe acyliert werden.
Diese Acylierung kann in inerten Lösungs- beziehungsweise Suspensionsmitteln wie Dioxan, Dimethylformamid, Benzol, Toluol, bei Temperaturen zwischen 0 bis 200° C, vorzugsweise 20 bis 150° C erfolgen. Als Acylierungsmittel kommen in Betracht: Ketene sowie Säurehalogenide (Chloride, Bromide, Jodide), Säureanhydride oder Säureester aliphatischer Carbonsäuren mit 2 - 13 C-Atomen, gegebenenfalls unter Zusatz eines säurebindenden Mittels wie Alkalicarbonaten, Alkalihydroxyden, Alkalialkoholaten oder eines tertiären Amins, zum Beispiel Triäthylamin oder Pyridin. Pyridin kann gleichzeitig auch als Lösungsmittel verwendet werden. Bei den Estern handelt es sich insbesondere um solche der obengenannten Carbonsäuren mit niederen aliphatischen Alkoholen. Bei der Acylierung kann man auch so vorgehen, daß man erst von der umzusetzenden Verbindung eine Alkaliverbindung herstellt,

...

indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) bei Temperaturen zwischen 0 und 150° C umsetzt und dann das acylierende Agens zufügt.

Die $C_2$-$C_{13}$-Alkanoylgruppen in den Verbindungen der Formel I können solvolytisch wieder abgespalten werden, wodurch die entsprechenden freien Hydroxy-Verbindungen der Formel I erhalten werden. Diese solvolytische Abspaltung erfolgt beispielsweise durch Verseifung mit verdünnten Säuren oder mittels basischer Substanzen (Pottasche, Soda, wässrige Alkalilösungen, alkoholische Alkalilösungen, $NH_3$) bei Temperaturen zwischen 10 und 150° C, insbesondere 20 - 100° C.

Als Lösungs- beziehungsweise Suspensionsmittel kommen hierfür beispielsweise in Betracht: Wasser, niedere aliphatische Alkohole, cyclische Äther wie Dioxan oder Tetrahydrofuran, aliphatische Äther, Dimethylformamid und so weiter sowie Mischungen dieser Mittel.

Die Herstellung der erfindungsgemäßen Verbindungen erfolgt nach dem Verfahren, das durch den Verfahrensanspruch gekennzeichnet ist.

...

Zu dem Verfahren:

Das Herstellungsverfahren wird im allgemeinen in einem inerten Lösungs- beziehungsweise Suspensionsmittel bei Temperaturen zwischen 5 und 250° C, vorzugsweise 20 - 150° C, insbesondere 40 - 90° C durchgeführt. Als Lösungsmittel kommen beispielsweise in Betracht: Niedere aliphatische Alkohole (Ethanol, Methanol, Isopropanol, Propanol), gesättigte alicyclische und cyclische Äther (Dioxan, Tetrahydrofuran, Diäthyläther), niedere aliphatische Ketone (Aceton), niedere aliphatische Kohlenwasserstoffe oder Halogenkohlenwasserstoffe (Benzol, Xylol, Toluol), Eisessig, Wasser beziehungsweise Mischungen dieser Mittel.

Der pH-Wert der Reaktionslösung beziehungsweise der Reaktionsmischung soll beispielsweise zwischen 1 - 6, vorzugsweise 2 - 3 liegen.

Falls als Ausgangsstoff eine Verbindung der Formel III verwendet wird, worin E die Gruppe $>CH_2$ ist, wird meist im unteren Teil des angegebenen Temperaturbereichs gearbeitet (5 bis 80° C), wobei hier als Lösungsmittel insbesondere niedere Alkohole, Äther, Aceton, Dioxan oder Chloroform in Betracht kommen. Bei Verwendung einer Verbindung III, worin E Wasserstoff und die Gruppe $-CH_2-NR_aR_b$ ist, wird meist in einem höheren Temperaturbereich (80 bis 250° C, insbesondere 80 bis 150° C) gearbeitet, wobei als Lösungsmittel insbesondere Wasser Alkohol/Wasser oder ein Zweiphasensystem wie Wasser/Benzol oder Wasser/Toluol verwendet wird.

Falls ein Ausgangsstoff der Formel III verwendet wird, wo E zwei Wasserstoffatome bedeutet, wird insbesondere zwischen 20 und 150° C gearbeitet. Als formaldehyd-

...

liefernde Substanzen kommen beispielsweise Formaldehydacetale in Betracht, aus denen in Gegenwart von Säuren Formaldehyd freigesetzt wird, wie zum Beispiel Polyformaldehyd, Paraformaldehyd, Hexamethylentetramin. Anstelle von Formaldehyd und dem Amin der Formel II kann auch ein zuvor aus diesen Stoffen hergestelltes Oxazolidin der Formel

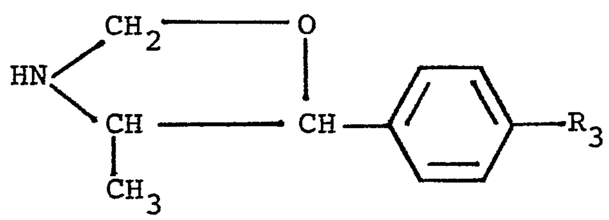

beziehungsweise ein Mineralsäureadditionssalz (zum Beispiel Hydrochlorid, Hydrobromid) hiervon mit der Verbindung der Formel

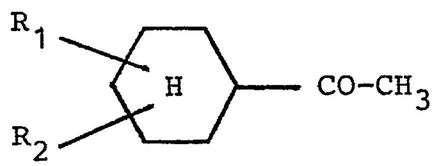

umgesetzt werden. Falls das Oxazolidinderivat nicht als Salz eingesetzt wird, ist es zweckmäßig, in Gegenwart einer Säure (zum Beispiel 20 %ige alkoholische Salzsäure oder Bromwasserstoffsäure) oder in Gegenwart eines sauren Ionenaustauschers zu arbeiten.

Anstelle des oben angegebenen Oxazolidins kann auch das entsprechende N,N'-Methylen-bis-oxazolidin der

...

folgenden Formel

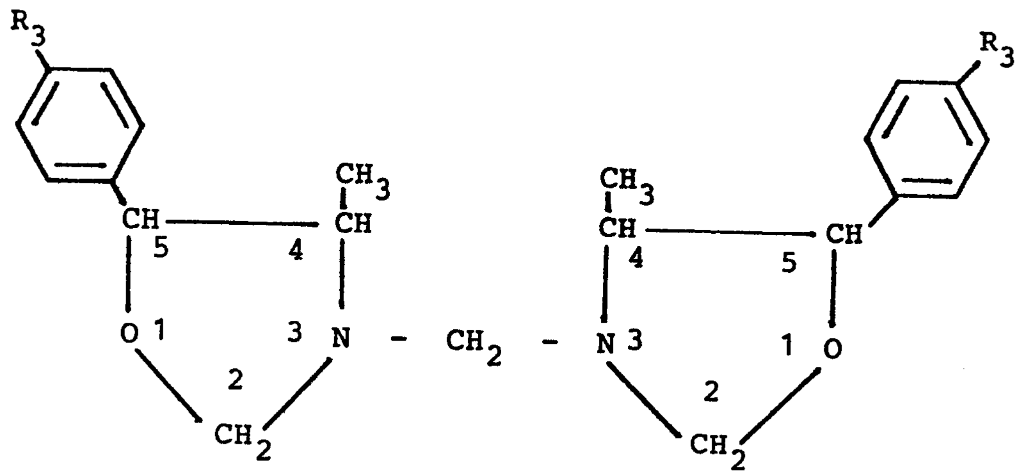

worin $R_3$ die angegebenen Bedeutungen hat, eingesetzt werden.

Im Falle der Verwendung eines N,N'-Methylen-bis-oxazolidins erfolgt das Verfahren beispielsweise durch Umsetzung von 1 Mol des Bis-oxazolidins mit 2 - 3 Mol, insbesondere 2,3 - 2,5 Mol des cycloaliphatischen Ketons in Gegenwart einer Säure oder eines sauren Ionenaustauschers in einem Lösungsmittel, vorzugsweise bei Temperaturen zwischen 20 bis 150° C.

Als Säuren kommen für das erfindungsgemäße Verfahren insbesondere Mineralsäuren, wie Salzsäure, Bromwasserstoffsäure, Schwefelsäure, Phosphorsäure in Frage. Jedoch können auch organische Säuren oder saure Ionenaustauscher verwendet werden. Als organische Säuren kommen beispielsweise in Frage: Gesättigte und einfach ungesättigte $C_1$-$C_4$-aliphatische Mono- und Dicarbonsäuren

...

wie Ameisensäure, Essigsäure, Propionsäure, Oxalsäure, Maleinsäure, Fumarsäure; aromatische Carbonsäuren wie Benzoesäure, $C_1$-$C_4$-Alkylbenzoesäuren oder aliphatische beziehungsweise aromatische Sulfonsäuren wie Methansulfonsäure, Ethansulfonsäure, Benzolsulfonsäure, Toluolsulfonsäure (zum Beispiel p-Toluolsulfonsäure). Es können auch Mischungen dieser Säuren verwendet werden. Als saure Ionenaustauscher kommen zum Beispiel solche in Betracht, die in dem Buch von K. Dorfner, Ionenaustauscher, Verlag De Gruyter, 3. Auflage, 1970, in Band 1 des Werkes Ionenaustauscher von E. Helfferich, Verlag Chemie, 1959 oder in Römpps Chemie Lexikon Band 3, Seiten 1616-1618 (1976) erwähnt sind. Im einzelnen handelt es sich zum Beispiel um organische Ionenaustauscher, deren aktive, das heißt ionenbildende Gruppe die Carboxylgruppe oder eine organische saure Gruppe wie zum Beispiel die Sulfonsäuregruppe ($SO_3H$) oder Phosphorsäuregruppe ist, während das hochmolekulare Gerüst (Matrix) aus einem Kunstharz (Acrylharz, Polystyrolharz, Styrol-Divinylbenzol-Copolymerisat, Styrol-Acrylsäure-Copolymerisat, Divinylbenzol-Polymerisat, Vinylbenzol-Polymerisat, Kondensationsprodukte aus Phenol und Formaldehyd) besteht. Weiterhin kommen in Betracht: saure Cellulose-, Stärke- und Dextran-Ionenaustauscher, Kohle-Ionenaustauscher (aus sulfonierten hochmolekularen Humuskohlen) sowie saure anorganische Ionenaustauscher wie Zeolithe und Aluminiumsilikate.

Die Herstellung der oben angegebenen Mono-Oxazolidine und Bis-Oxazolidine ist bekannt.

Die Ausgangsstoffe der Formel III, worin die Gruppe -CH=E die Methylgruppe ($-CH_3$) ist, können zum Beispiel nach der von H. Stetter und R. Hesse, Monatshefte Chemie 98, 755 ff. (1967) beschriebenen Methode aus

...

den entsprechenden Mono- oder Dihydroxy-cyclohexancarbonsäureethylestern hergestellt werden. Hierbei werden diese Cyclohexancarbonsäureethylester zuerst mit Phenylsulfon-methylmagnesiumbromid umgesetzt und anschließend in den so erhaltenen Reaktionsprodukten mit Aluminium-amalgam die Phenylsulfongruppe abgespalten. Die Abspaltung der Phenylsulfongruppe kann zum Beispiel gemäß der folgenden allgemeinen Vorschrift erfolgen:

0,11 Mol des im Cyclohexanring entsprechend mono- oder dihydroxylierten 1-Phenylsulfon-2-oxo-2-cyclohexyl-äthans werden in 1200 ml Tetrahydrofuran gelöst, dem man 10 % $H_2O$ zugesetzt hat. Zu diesem Gemisch werden 30 g Aluminium-Amalgam zugegeben. Nach beendeter Zugabe rührt man 30 Minuten nach und kocht anschließend 75 Minuten am Rückfluß. Nach Abkühlen der Lösung wird diese im Vakuum eingeengt. Das nach Einengen erhaltene kristalline Produkt wird nun 6 mal mit je 100 ml Ether extrahiert. Die vereinigten Etherextrakte werden über $MgSo_4$ getrocknet, filtriert und dann eingeengt. Das so erhaltene farblöse Öl wird dann im Vakuum fraktioniert destilliert.

...

Ausgangsstoffe der Formel III, worin die Gruppe -CH=E die Gruppe $-CH_2-CH_2-NR_aR_b$ ist, können zum Beispiel durch Mannichreaktion aus Ketonen der Formel III, worin -CH=E die Methylgruppe ist, mit einem niederen Dialkylamin oder einem Cycloalkylamin in Anwesenheit von Formaldehyd erhalten werden. Ausgangsstoffe der Formel III, worin -CH=E die Gruppe $-CH=CH_2$ ist, können zum Beispiel aus Ketonen der Formel III, worin -CH=E die Gruppe $-CH_2-CH_2NR_aR_b$ ist, durch Abspaltung des Aminteils (zum Beispiel durch Wasserdampfdestillation) oder durch thermische HCl-Abspaltung aus den entsprechenden cycloaliphatischen ß-Chlor-äthylketonen (Formel III $-CH=E = -CH_2-CH_2Cl$) erhalten werden.

Diejenigen Verbindungen, die asymmetrische Kohlenstoffatome enthalten und in der Regel als Racemate anfallen, können in an sich bekannter Weise, zum Beispiel mittels einer optisch aktiven Säure in die optisch aktiven Isomeren gespalten werden. Es ist aber auch möglich, von vornherein optisch aktive beziehungsweise auch diastereomere Ausgangsstoffe einzusetzen, wobei dann als Endprodukt eine entsprechende reine optisch aktive Form beziehungsweise diastereomere Konfiguration erhalten wird. Beispielsweise handelt es sich um Verbindungen der Norephedrin- und der Pseudonorephedrin-Konfiguration. Es können auch diastereomere Racemate auftreten, da in den hergestellten Verbindungen zwei oder mehr asymmetrische Kohlenstoffatome vorhanden sind. Trennung ist auf üblichem Weg, zum Beispiel durch Umkristallisieren, Chromatographie sowie die sonstigen üblichen Verfahren zur Diastereomerentrennung möglich. Im allgemeinen werden die Verfahrensprodukte infolge des substituierten Cyclohexanringes als stereoisomere Gemische erhalten. Das gemäß Beispiel 1 erhaltene Verfahrensprodukt liegt beispielsweise zu 87,5 % in der trans-Form und zu 12,5 % in der cis-Form vor. Das gemäß Beispiel 2 erhaltene Verfahrensprodukt liegt zu 66,6 % in der trans-Form und zu 33,3 % in der cis-Form vor.

...

den entsprechenden Mono- oder Dihydroxy-cyclohexancarbonsäureethylestern hergestellt werden. Hierbei werden diese Cyclohexancarbonsäureethylester zuerst mit Phenylsulfon-methylmagnesiumbromid umgesetzt und anschließend in den so erhaltenen Reaktionsprodukten mit Aluminium-amalgam die Phenylsulfongruppe abgespalten. Die Abspaltung der Phenylsulfongruppe kann zum Beispiel gemäß der folgenden allgemeinen Vorschrift erfolgen:

0,11 Mol des im Cyclohexanring entsprechend mono- oder dihydroxylierten 1-Phenylsulfon-2-oxo-2-cyclohexyl-äthans werden in 1200 ml Tetrahydrofuran gelöst, dem man 10 % $H_2O$ zugesetzt hat. Zu diesem Gemisch werden 30 g Aluminium-Amalgam zugegeben. Nach beendeter Zugabe rührt man 30 Minuten nach und kocht anschließend 75 Minuten am Rückfluß. Nach Abkühlen der Lösung wird diese im Vakuum eingeengt. Das nach Einengen erhaltene kristalline Produkt wird nun 6 mal mit je 100 ml Ether extrahiert. Die vereinigten Etherextrakte werden über $MgSo_4$ getrocknet, filtriert und dann eingeengt. Das so erhaltene farblöse Öl wird dann im Vakuum fraktioniert destilliert.

...

Ausgangsstoffe der Formel III, worin die Gruppe -CH=E die Gruppe $-CH_2-CH_2-NR_aR_b$ ist, können zum Beispiel durch Mannichreaktion aus Ketonen der Formel III, worin -CH=E die Methylgruppe ist, mit einem niederen Dialkylamin oder einem Cycloalkylamin in Anwesenheit von Formaldehyd erhalten werden. Ausgangsstoffe der Formel III, worin -CH=E die Gruppe $-CH=CH_2$ ist, können zum Beispiel aus Ketonen der Formel III, worin -CH=E die Gruppe $-CH_2-CH_2NR_aR_b$ ist, durch Abspaltung des Aminteils (zum Beispiel durch Wasserdampfdestillation) oder durch thermische HCl-Abspaltung aus den entsprechenden cycloaliphatischen ß-Chlor-äthylketonen (Formel III $-CH=E = -CH_2-CH_2Cl$) erhalten werden.

Diejenigen Verbindungen, die asymmetrische Kohlenstoffatome enthalten und in der Regel als Racemate anfallen, können in an sich bekannter Weise, zum Beispiel mittels einer optisch aktiven Säure in die optisch aktiven Isomeren gespalten werden. Es ist aber auch möglich, von vornherein optisch aktive beziehungsweise auch diastereomere Ausgangsstoffe einzusetzen, wobei dann als Endprodukt eine entsprechende reine optisch aktive Form beziehungsweise diastereomere Konfiguration erhalten wird. Beispielsweise handelt es sich um Verbindungen der Norephedrin- und der Pseudonorephedrin-Konfiguration. Es können auch diastereomere Racemate auftreten, da in den hergestellten Verbindungen zwei oder mehr asymmetrische Kohlenstoffatome vorhanden sind. Trennung ist auf üblichem Weg, zum Beispiel durch Umkristallisieren, Chromatographie sowie die sonstigen üblichen Verfahren zur Diastereomerentrennung möglich. Im allgemeinen werden die Verfahrensprodukte infolge des substituierten Cyclohexanringes als stereoisomere Gemische erhalten. Das gemäß Beispiel 1 erhaltene Verfahrensprodukt liegt beispielsweise zu 87,5 % in der trans-Form und zu 12,5 % in der cis-Form vor. Das gemäß Beispiel 2 erhaltene Verfahrensprodukt liegt zu 66,6 % in der trans-Form und zu 33,3 % in der cis-Form vor.

...

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern, wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen oder auch Mischungen derselben mit anderen pharmazeutisch wirksamen Stoffen enthalten. Zur Herstellung der pharmazeutischen Zubereitungen können die üblichen pharmazeutischen Träger und Hilfsstoffe verwendet werden. Die Arzneimittel können enteral, parenteral, oral oder perlingual angewendet werden. Beispielsweise kann die Verabreichung in Form von Tabletten, Kapseln, Dragees, Zäpfchen, Salben, Puder, Liquida oder Aerosolen erfolgen. Als Liquida kommen zum Beispiel in Frage: ölige oder wässrige Lösungen oder Suspensionen, Emulsionen, injizierbare wässrige oder ölige Lösungen oder Suspensionen.

In den Beispielen wird jeweils vom $\ell$-Norephedrin ausgegangen. Die entsprechenden d-Isomere beziehungsweise das Racemat erhält man, wenn beispielsweise anstelle der linksdrehenden Norephedrin-Ausgangsverbindung die entsprechende rechtsdrehende Form beziehungsweise das Racemat verwendet wird.

...

Manchmal empfiehlt es sich, zur Isolierung des Verfahrensproduktes das meist als Hydrochlorid anfallende unmittelbare Reaktionsprodukt durch Behandeln mit beispielsweise verdünntem Ammoniak in die freie Base zu überführen und gegebenenfalls dieselbe in üblicher Weise erneut in ein Salz zu überführen.

...

Die erfindungsgemäßen Verbindungen zeigen am narkotisierten Hund (Narkose mit Pentobarbital) zum Beispiel die folgende Wirkung:
Die Kontraktilität des Herzmuskels wird gesteigert, das Herzzeitvolumen verbessert und der Blutdurchfluß in den Arterien erhöht.

Beispielsweise wird bei obengenannter Versuchsmethode bei einer Dosis von 0,3 mg/kg Körpergewicht (Hund) eine Inotropiezunahme von 26 % erzielt. Die niedrigste, bereits wirksame Dosis in dem obengenannten Tierversuch ist beispielsweise 0,1 mg/kg intravenös.

Als allgemeiner Dosisbereich für die Wirkung (Tierversuch wie oben) kommt beispielsweise in Frage: 0,1 - 1,0 mg/kg intravenös, insbesondere 0,3 mg/kg.

Indikationen, für die die erfindungsgemäßen Verbindungen in Betracht kommen können: beispielsweise Myokardinsuffizienz.

Die pharmazeutischen Zubereitungen enthalten im allgemeinen zwischen 1 bis 50 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 5 und 50 mg oder Lösungen, die zwischen 0,1 bis 10 % an aktiver Substanz enthalten.

...

Die Einzeldosis der erfindungsgemäßen aktiven Komponenten kann beispielsweise liegen

a) bei oralen Arzneiformen zwischen 5 und 100 mg

b) bei parenteralen Arzneiformen (zum Beispiel intravenös, intramuskulär) zwischen 0,5 und 20 mg.

Beispielsweise können 3 mal täglich 1 bis 3 Tabletten mit einem Gehalt von 10 bis 20 mg wirksamer Substanz oder zum Beispiel bei intravenöser Injektion 1 bis 5 mal täglich eine Ampulle von 2 bis 10 ml Inhalt mit 5 bis 15 mg Substanz empfohlen werden. Bei oraler Verabreichung ist die minimale tägliche Dosis beispielsweise 10 mg; die maximale tägliche Dosis bei oraler Verabreichung soll nicht über 300 mg liegen.

Für die Behandlung von Hunden und Katzen liegt die orale Einzeldosis im allgemeinen zwischen ungefähr 0,3 und 5 mg/kg Körpergewicht; die parenterale Dosis ungefähr zwischen 0,1 und 10 mg/kg Körpergewicht.

- Die Dosen sind jeweils bezogen auf die freie Base -

...

0211254

Für die Behandlung von Pferden und Vieh liegt die orale Einzeldosis im allgemeinen zwischen ungefähr 0,3 und 5 mg/kg; die parenterale Einzeldosis ungefähr zwischen 0,1 und 10 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation oberhalb 250 mg/kg (zum Beispiel zwischen 250 und 600 mg/kg).

Die Arzneimittel können in der Humanmedizin, der Veterinärmedizin sowie in der Landwirtschaft allein oder im Gemisch mit anderen pharmakologisch aktiven Stoffen verwendet werden.

...

Beispiele

Beispiel 1

ℓ-[3-Hydroxy-3-phenyl-propyl-(2)]-[3-(4-hydroxy-cyclohexyl)-3-oxo-propyl]-amin

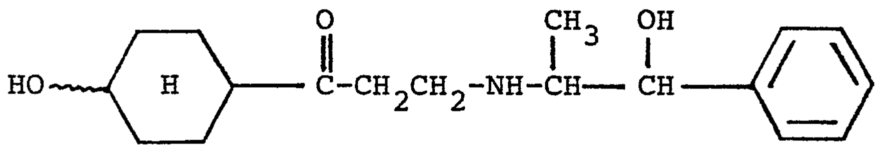

Die Reaktionsmischung aus 5 g (0,03 Mol) 4-Hydroxy-1-acetyl-cyclohexan ($Kp_3$ 105-106° C), 5,9 g (0,018 Mol) ℓ-N,N'-Methylen-bis-(4-methyl-5-phenyl)-oxazolidin (hergestellt aus ℓ-Norephedrin), 40 ml Isopropanol und 8 ml 5,1 normale isopropanolischer Salzsäure wird 3 Stunden unter Rühren und Rückfluß erhitzt. Die Reaktionsmischung wird über Nacht bei Raumtemperatur belassen. Das auskristallisierte Produkt wird abgenutscht und mit Isopropanol (5 ml) und 10 ml Aceton gewaschen.
F. des Hydrochlorids: 196 - 199° C.

...

Beispiel 2

ℓ-/3-Hydroxy-3-phenyl-propyl-(2)7-/3-(3-hydroxy-cyclohexyl)-3-oxo-propyl7-amin

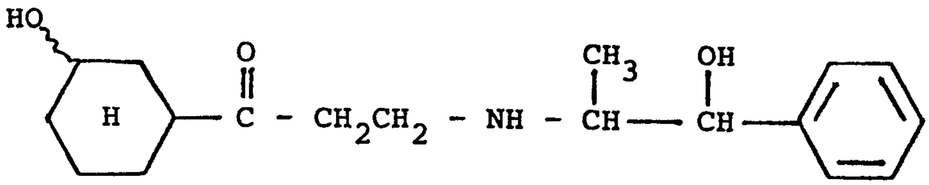

5 g (0,03 Mol) 3-Hydroxy-1-acetyl-cyclohexan ($Kp_4$ 116 - 177° C), 5,9 g (0,018 Mol) ℓ-N,N'-Methylen-bis-(4-methyl-5-phenyl)-oxazolidin (hergestellt aus ℓ-Norephedrin), 40 ml Isopropanol und 8 ml 5,1 normale isopropanolische Salzsäure werden 6 Stunden am Rückfluß erhitzt. Nach beendetem Rückflußkochen wird das abgekühlte Reaktionsgemisch im Vakuum eingeengt. Der eingeengte Rückstand wird über Kieselgel chromatographiert. Als Laufmittel dient Dichlormethan/Methanol im Verhältnis 95:5. Das chromatographierte Produkt wird durch Versetzen mit isopropanolischer HCl ins Hydrochlorid überführt.
F. des Hydrochlorids: 177 - 179° C.

Beispiel 3

ℓ-[3-Hydroxy-3-phenyl-propyl-(2)]-[3-(3,4-dihydroxy-cyclohexyl)-3-oxo-propyl]-amin

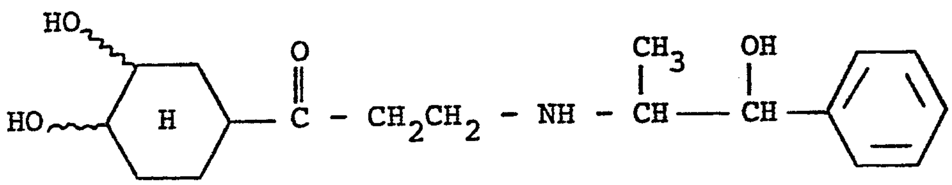

4,6 g (0,03 Mol) 3,4-Dihydroxy-1-acetyl-cyclohexan, 5,9 g (0,018 Mol) ℓ-N,N'-Methylen-bis-(4-methyl-5-phenyl)-oxazolidin (hergestellt aus ℓ-Norephedrin), 40 ml Isopropanol und 8 ml 5,1 normale isopropanolische Salzsäure werden 6 Stunden am Rückfluß erhitzt. Danach läßt man abkühlen und engt das erhaltene Reaktionsgemisch im Vakuum ein. Der eingeengte Rückstand wird über Kieselgel chromatographiert. Als Laufmittel wird Dichlormethan/Methanol im Verhältnis 90:10 verwendet. Das chromatographierte Produkt wird ins Hydrochlorid überführt.
F. des Hydrochlorids: 178 - 181° C. Kernmagnetisches Resonanzspektrum in deuteriertem Chloroform bei 60 Megahertz (chemische Verschiebung ($\delta$) der Wasserstoffatome mit Spin 1/2):

$\delta$ = 1,1 (Duplet der 3 H der $C\underline{H}_3$-Gruppe)
$\delta$ = 1,2 - 2,2 (Multiplet der 6 H der 3-Ring-$C\underline{H}_2$-Gruppen)
$\delta$ = 2,4 - 2,6 (Multiplet des H des $>C\underline{H}$-CO-Teils)
$\delta$ = 3,0 - 4,0 (Multiplet der 7 H der Strukturteile $-CO-C\underline{H}_2-C\underline{H}_2-NH$; $-NH-C\underline{H}-CH_3$; 2 mal $>C\underline{H}\sim OH$)

...

= 5,2 (Duplet 1 H des Strukturteils $-\overset{OH}{\overset{|}{C}}\underline{H}-$)

= 7,4 (Multiplet 5 H des Phenylringes).

Beispiel 4

ℓ-/3-Hydroxy-3-(4-benzyloxyphenyl)-propyl-(2)7-/3-(3-hydroxy-cyclohexyl)-3-oxo-propyl-(1)7-amin

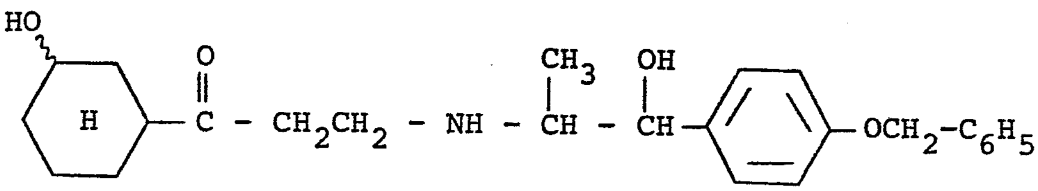

1,42 g (0,01 Mol) 3-Hydroxy-1-acetyl-cyclohexan werden mit 2,8 g ℓ-N,N'-Methylen-bis-/4-methyl-5-(4-benzyloxy-phenyl)-oxazolidin (hergestellt aus ℓ-4-Hydroxynorephedrin), 5 ml 5,1 normale isopropanolische Salzsäure und 40 ml Isopropanol 6 Stunden bei 70° C erhitzt. Danach läßt man abkühlen und engt das erhaltene Reaktionsgemisch im Vakuum ein. Der Rückstand wird mit wässrigem Ammoniak neutralisiert und das erhaltene Produkt über Kieselgel chromatographiert. Als Laufmittel wird Dichlormethan/Methanol im Verhältnis 85:15 verwendet. Das erhaltene Produkt wird in Aceton in das Oxalsäuresalz überführt.

F. des Oxalats: 123 - 126° C

Beispiel 5 (Abspaltung einer Schutzgruppe)

ℓ-[3-Hydroxy-3-(4-hydroxyphenyl)-propyl-(2)]-[3-(3-hydroxy-cyclohexyl)-3-oxo-propyl]-amin

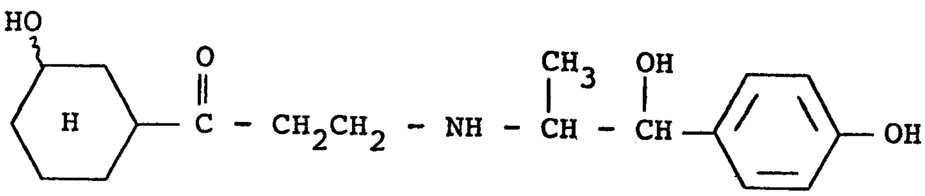

90 mg (0,0002 Mol) ℓ-[3-Hydroxy-3-(4-benzyloxyphenyl)-propyl-(2)]-[3-(3-hydroxy-cyclohexyl)-3-oxo-propyl-(1)]-amin-oxalat werden in Methanol gelöst und mit 9 mg Pd-C (10 %ig) versetzt. Es wird 1 Stunde bei 1 bar-Druck hydriert, der Katalysator abgesaugt und die Lösung eingeengt. Das erhaltene Öl wird mit Diethylether gerührt, die resultierenden Kristalle abgesaugt und aus Isopropanol/Methanol umkristallisiert.

F. des Oxalats: 195 - 197° C.

0211254

## Beispiele für pharmazeutische Zubereitungen

### Beispiel für Tabletten

Für einen Ansatz von 100 000 Tabletten werden benötigt:

| | |
|---|---|
| 1. Verbindung gemäß Beispiel 1 (Hydrochlorid) | 5,00 kg |
| 2. Magnesiumstearat | 0,05 kg |
| 3. Lactose | 6,20 kg |
| 4. mikrokristalline Cellulose | 6,70 kg |
| 5. Maisstärke | 1,00 kg |
| 6. Formalin Kasein | 1,00 kg |
| 7. Hochdisperse Kieselsäure | 0,05 kg |
| Gewicht der Tabletten-Kerne: | 20,00 kg |

Herstellung der Tabletten:

1. 3,5 kg mikrokristalline Cellulose werden mit 0,4 Liter entmineralisiertem Wasser angefeuchtet.

2. Die angefeuchtete mikrokristalline Cellulose wird mit den Substanzen 2, 5, 6 und 7 in einem geeigneten Mischer 5 Minuten lang gemischt.
   = Mischung 1

3. Mischung 1 sowie die Substanzen 1, 3 und die restliche mikrokristalline Cellulose werden im Anschluß daran gesiebt (Sieb 0,8 - 1 mm Maschenweite) und in einem geeigneten Mischer homogen gemischt.
   = Mischung 2
   = Preßmasse

...

0211254

4. Die relative Feuchtigkeit der Preßmasse muß im Bereich von 40 - 45 % liegen.

5. Die Preßmasse wird anschließend auf einer Rundlaufpresse zu gewölbten Tabletten mit folgenden Merkmalen verpreßt:

| | |
|---|---|
| Gewicht: | 200 mg |
| Durchmesser: | 8 mm |
| Wölbungsradius: | 8 mm |
| Härte: | 5 - 7 kg (Monsanto-Härtetester) |
| Zerfallzeit in kaltem Wasser: | maximal 5 Minuten |

Beispiel für eine Injektionslösung:

Für einen Ansatz von 100 Liter Injektionslösung werden benötigt:

| | | |
|---|---|---|
| 1. | Verbindung gemäß Beispiel 1 (Hydrochlorid) | 0,90 kg |
| 2. | Äthanol 96 % | 10,00 kg |
| 3. | 1,2-Propylenglykol | 25,00 kg |
| 4. | Wasser für Injektionszwecke ad | 100 Liter |

Herstellung der Injektionslösung:

1 wird in einer Mischung aus 2, 3 und 60 Liter Wasser für Injektionszwecke unter Rühren auf dem Wasserbad in Lösung gebracht. Nach dem Abkühlen der Lösung auf 20° C wird der pH-Wert gemessen und gegebenenfalls durch Zugabe von 1 N-HCl auf 2 ± 0,2 eingestellt. Danach wird die Lösung mit Wasser für Injektionszwecke auf ein Gesamtvolumen von 100 Liter gebracht.

= Wirkstofflösung

...

Die Wirkstofflösung wird steril filtriert, in farblose Ampullengläser à 2 ml abgefüllt und danach 20 Minuten bei 120° C sterilisiert.

1 Ampulle = 2 ml Injektionslösung enthält 18 mg Wirksubstanz.

4. Die relative Feuchtigkeit der Preßmasse muß im Bereich von 40 - 45 % liegen.

5. Die Preßmasse wird anschließend auf einer Rundlaufpresse zu gewölbten Tabletten mit folgenden Merkmalen verpreßt:

| | |
|---|---|
| Gewicht: | 200 mg |
| Durchmesser: | 8 mm |
| Wölbungsradius: | 8 mm |
| Härte: | 5 - 7 kg (Monsanto-Härtetester) |
| Zerfallzeit in kaltem Wasser: | maximal 5 Minuten |

Beispiel für eine Injektionslösung:

Für einen Ansatz von 100 Liter Injektionslösung werden benötigt:

| | | |
|---|---|---|
| 1. | Verbindung gemäß Beispiel 1 (Hydrochlorid) | 0,90 kg |
| 2. | Äthanol 96 % | 10,00 kg |
| 3. | 1,2-Propylenglykol | 25,00 kg |
| 4. | Wasser für Injektionszwecke ad | 100 Liter |

Herstellung der Injektionslösung:

1 wird in einer Mischung aus 2, 3 und 60 Liter Wasser für Injektionszwecke unter Rühren auf dem Wasserbad in Lösung gebracht. Nach dem Abkühlen der Lösung auf 20° C wird der pH-Wert gemessen und gegebenenfalls durch Zugabe von 1 N-HCl auf 2 $\pm$ 0,2 eingestellt. Danach wird die Lösung mit Wasser für Injektionszwecke auf ein Gesamtvolumen von 100 Liter gebracht.

= Wirkstofflösung

...

Die Wirkstofflösung wird steril filtriert, in farblose Ampullengläser à 2 ml abgefüllt und danach 20 Minuten bei 120° C sterilisiert.

1 Ampulle = 2 ml Injektionslösung enthält 18 mg Wirksubstanz.

0211254

Degussa Aktiengesellschaft
Weissfrauenstraße 9, 6000 Frankfurt/Main

Neue cycloaliphatische Ketoamine

Patentansprüche:

1. Verbindungen der Formel

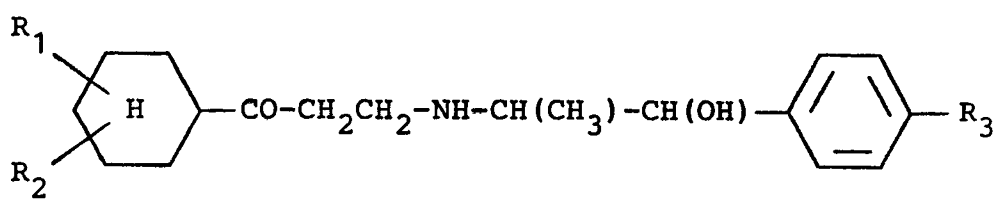

I

worin $R_1$ Wasserstoff, eine Hydroxygruppe oder eine $C_2$-$C_{13}$-Alkanoyloxygruppe, $R_2$ eine Hydroxygruppe oder eine $C_2$-$C_{13}$-Alkanoyloxygruppe und $R_3$ Wasserstoff, eine Hydroxygruppe, eine $C_2$-$C_{13}$-Alkanoyloxygruppe oder eine Benzyloxygruppe ist und deren Säureadditionssalze.

...

2. Verfahren zur Herstellung von Verbindungen der Formel

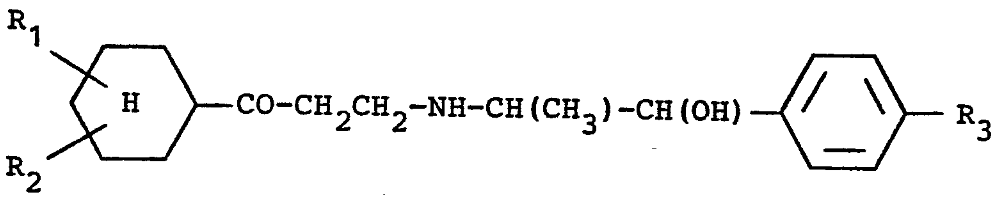

I

worin $R_1$ Wasserstoff, eine Hydroxygruppe oder eine $C_2$-$C_{13}$-Alkanoyloxygruppe, $R_2$ eine Hydroxygruppe oder eine $C_2$-$C_{13}$-Alkanoyloxygruppe und $R_3$ Wasserstoff, eine Hydroxygruppe, eine $C_2$-$C_{13}$-Alkanoyloxygruppe oder eine Benzyloxygruppe ist und deren Säureadditionssalze, dadurch gekennzeichnet,
daß man ein Amin der allgemeinen Formel

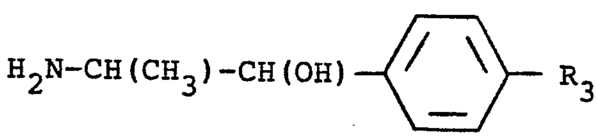

II

worin $R_3$ die angegebenen Bedeutungen hat, mit einer Verbindung der allgemeinen Formel

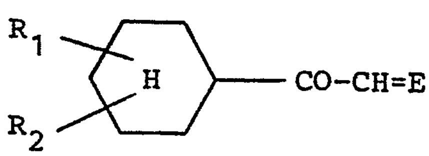

III

...

umsetzt, wobei $R_1$ und $R_2$ die angegebenen Bedeutungen haben und E eine Methylengruppe oder ein Wasserstoffatom plus die Gruppe $-CH_2-NR_aR_b$ ist und $R_a$ und $R_b$ niedrigmolekulare Alkylreste sind, die auch zu einem Ring geschlossen sein können, oder wobei E zwei Wasserstoffatome bedeutet, wenn in Gegenwart von Formaldehyd oder eines formaldehydliefernden Stoffes gearbeitet wird, gegebenenfalls die erhaltenen Verbindungen acyliert und/oder vorhandene Schutzgruppen abspaltet
und die erhaltenen Verbindungen gegebenenfalls in ihre Säureadditionssalze überführt.

3. Verbindungen der Formel I zur Anwendung als therapeutische Wirkstoffe.

4. Arzneimittel, enthaltend eine Verbindung der allgemeinen Formel I neben üblichen Träger- und/oder Verdünnungs- beziehungsweise Hilfsstoffen.

5. Verfahren zur Herstellung eines Arzneimittels, dadurch gekennzeichnet,
daß eine Verbindung der allgemeinen Formel I mit gebräuchlichen pharmazeutischen Trägerstoffen und/oder Verdünnungsmittels beziehungsweise sonstigen Hilfsstoffen zu pharmazeutischen Zubereitungen verarbeitet beziehungsweise in eine therapeutisch anwendbare Form gebracht wird.

...

6. Verwendung von Verbindungen der allgemeinen Formel I zur Herstellung von Arzneimitteln.

...

0211254

Patentansprüche für den benannten Vertragsstaat Österreich:

1. Verbindungen der Formel

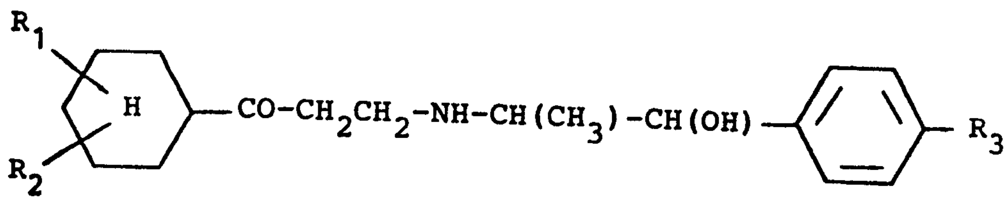

I

worin $R_1$ Wasserstoff, eine Hydroxygruppe oder eine $C_2$-$C_{13}$-Alkanoyloxygruppe, $R_2$ eine Hydroxygruppe oder eine $C_2$-$C_{13}$-Alkanoyloxygruppe und $R_3$ Wasserstoff, eine Hydroxygruppe, eine $C_2$-$C_{13}$-Alkanoyloxygruppe oder eine Benzyloxygruppe ist und deren Säureadditionssalze.

2. Verfahren zur Herstellung von Verbindungen der Formel

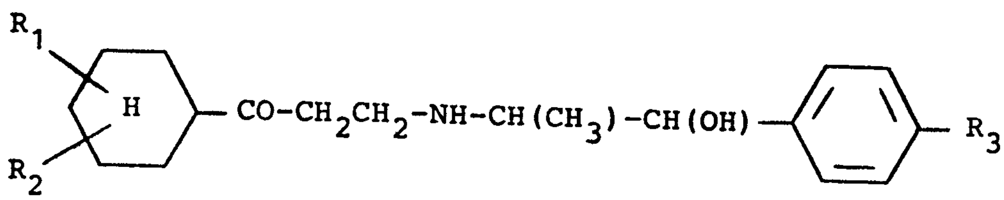

I

worin $R_1$ Wasserstoff, eine Hydroxygruppe oder eine $C_2$-$C_{13}$-Alkanoyloxygruppe, $R_2$ eine Hydroxygruppe

...

oder eine $C_2$-$C_{13}$-Alkanoyloxygruppe und $R_3$ Wasserstoff, eine Hydroxygruppe, eine $C_2$-$C_{13}$-Alkanoyloxygruppe oder eine Benzyloxygruppe ist und deren Säureadditionssalze, dadurch gekennzeichnet,

daß man ein Amin der allgemeinen Formel

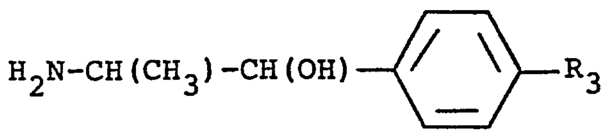

II

worin $R_3$ die angegebenen Bedeutungen hat, mit einer Verbindung der allgemeinen Formel

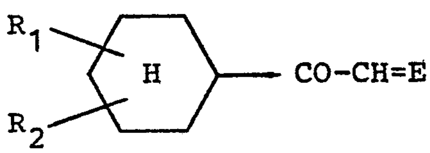

III

umsetzt, wobei $R_1$ und $R_2$ die angegebenen Bedeutungen haben und E eine Methylengruppe oder ein Wasserstoffatom plus die Gruppe $-CH_2-NR_aR_b$ ist und $R_a$ und $R_b$ niedrigmolekulare Alkylreste sind, die auch zu einem Ring geschlossen sein können, oder wobei E zwei Wasserstoffatome bedeutet, wenn in Gegenwart von Formaldehyd oder eines formaldehydliefernden Stoffes gearbeitet wird, gegebenenfalls die erhaltenen Verbindungen acyliert und/oder vorhandene Schutzgruppen abspaltet

und die erhaltenen Verbindungen gegebenenfalls in ihre Säureadditionssalze überführt.

...

3. Verbindungen der Formel I zur Anwendung als therapeutische Wirkstoffe.

4. Arzneimittel, enthaltend eine Verbindung der allgemeinen Formel I neben üblichen Träger- und/oder Verdünnungs- beziehungsweise Hilfsstoffen.

5. Verfahren zur Herstellung eines Arzneimittels, dadurch gekennzeichnet, daß eine Verbindung der allgemeinen Formel I mit gebräuchlichen pharmazeutischen Trägerstoffen und/oder Verdünnungsmittels beziehungsweise sonstigen Hilfsstoffen zu pharmazeutischen Zubereitungen verarbeitet beziehungsweise in eine therapeutisch anwendbare Form gebracht wird.

6. Verfahren zur Herstellung eines (herzwirksamen) Arzneimittels, dadurch gekennzeichnet, daß man Verbindungen der Fomel I beziehungsweise deren Salze mit physiologisch unbedenklichen Säuren zusammen mit üblichen Träger- und/oder Verdünnungs- beziehungsweise Hilfsstoffen bei Temperaturen zwischen 20 und 100° C vermischt beziehungsweise homogenisiert, die so erhaltene Mischung zur Herstellung von Zubereitungen, die in der Dosierungseinheit 1 bis 50 mg Wirkstoff der Formel I enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

...

7. Verfahren zur Herstellung eines (herzwirksamen) Arzneimittels, dadurch gekennzeichnet, daß man Verbindungen der Formel I beziehungsweise deren Salze mit physiologisch unbedenklichen Säuren mit einem oder mehreren der folgenden Stoffe: Maisstärke, mikrokristalline Cellulose, Lactose, Formalinkasein, modifizierte Stärke, Magnesiumstearat, hochdisperse Kieselsäure vermischt und die erhaltene Mischung, gegebenenfalls nach Granulierung mit einer Gelatinelösung zu Tabletten verpresst, die in der Dosierungseinheit 1 bis 50 mg Wirkstoff der Formel I enthalten.

8. Verfahren zur Herstellung eines (herzwirksamen) Arzneimittels, dadurch gekennzeichnet, daß man Verbindungen der Formel I oder deren Salze mit physiologisch unbedenklichen Säuren nach Zusatz von Sojalecithin bei Temperaturen zwischen 33 - 37° C in geschmolzenem Hartfett suspendiert und homogenisiert und anschließend die Mischung in Hohlzellen ausgießt, wobei die Dosierungseinheit 1 bis 50 mg Wirkstoff enthält.

9. Verfahren zur Herstellung einer herzwirksamen Injektionslösung, dadurch gekennzeichnet, daß man Verbindungen der Formel I oder deren Salze mit physiologisch unbedenklichen Säuren in Wasser bei Temperaturen zwischen 80 - 100° C auflöst, nach Abkühlen gegebenenfalls mittels 1 N-HCl einen pH-Wert von 2 $\pm$ 0,2 einstellt, und die so erhaltene

...

0211254

Lösung mit soviel Wasser auffüllt, daß die Endlösung 0,1 bis 10 Gewichtsprozent an Wirkstoff der Formel I enthält.

10. Verwendung von Verbindungen der allgemeinen Formel I zur Herstellung von Arzneimitteln.